# EUROPÄISCHE PATENTSCHRIFT

(11) **EP 2 363 350 B1**
(45) Veröffentlichungstag und Bekanntmachung des Hinweises auf die Patenterteilung: **10.10.2012**
(21) Anmeldenummer: 10405191.7
(22) Anmeldetag: 12.10.2010
(51) Int. Cl.: B65D 41/34, A61B 10/00

(54) **Behälter für eine fälschungssichere Aufbewahrung**
Container for forgery-proof storage
Récipient destiné à un stockage protégé contre la contrefaçon

(30) Priorität: 01.03.2010 CH 2542010
(43) Veröffentlichungstag der Anmeldung: 07.09.2011
(73) Patentinhaber: Berlinger & Co., 9608 Ganterschwil (CH)
(72) Erfinder: Oertli, Christian, 8620 Wetzikon (CH); Schaufelberger, Martin, 8872 Weesen (CH)
(74) Vertreter: Quehl, Horst Max

(56) Entgegenhaltungen:
- DE-U1- 29 610 161
- FR-A1- 2 779 702
- GB-A- 2 102 774

## Beschreibung

Die Erfindung betrifft einen Behälter mit einem Verschlusssystem für eine fälschungssichere Aufbewahrung entsprechend den Merkmalen des Oberbegriff des Patentanspruchs 1.

Ein Behälter dieser Art is bekannt durch die DE 29610161 U1. Bei diesem wird beim erstmaligem Entfernen der Verschlusskappe ein an einem Sicherungsring vorgesehener Solltrennbereich zerstört, so dass der Sicherungsring nach Wiederverschliessen des Behälters seine Funktion für eine fälschungssichere Aufbewahrung verliert.

Durch die DE 4318311 ist weiterhin ein Behälter bekannt, an dem zwischen ringförmig angeordneten, zahnförmigen Verriegelungsmitteln eine ringförmige Schutzabdeckung vorgesehen, so dass diese vor dem Schliessen des Behälters zu entfernen ist. Um eine Erstverwendung dieses Behälters zu garantieren bzw. sein unkontrolliertes Füllen zu verhindern, ist zumindest sein oberer Bereich zusammen mit seiner Verschlussvorrichtung in einer gemeinsamen, z.B. durch eine Schrumpffolie gebildete Ummantelung fest eingeschlossen, die entlang eines Solltrennbereichs aufreissbar ist.

Der Erfindung liegt die Aufgabe zugrunde, einen Behälter der eingangs genannten Art zu finden der mit erhöhter Sicherheit seine Aufgabe erfüllt und der ausserdem kostengünstig herstellbar ist.

Die Lösung der genannten Aufgabe erfolgt erfindungsgemäss aufgrund der kennzeichnenden Merkmale des Patentanspruchs 1. Vorteilhafte Ausgestaltungen der Erfindung sind Gegenstand der abhängigen Patentansprüche und der folgenden Beschreibung anhand der Zeichnungen zu entnehmen.

Es zeigt:
Fig.1 einen Axialschnitt durch einen erfindungsgemässen Behälter in verschlossenem Zustand, vor seiner Verwendung,
Fig.2 eine perspektivische Darstellung der in einem Behälter entsprechend Fig.1 zusammengefügten Einzelteile,
Fig.3 eine perspektivische Darstellung eines Klinkenringes in Eingriff mit einem Sicherungsring der Verschlusskappe des Behälters nach Fig.1,
Fig.4 eine Ansicht der Unterseite des Sicherungsringes nach Fig.3,

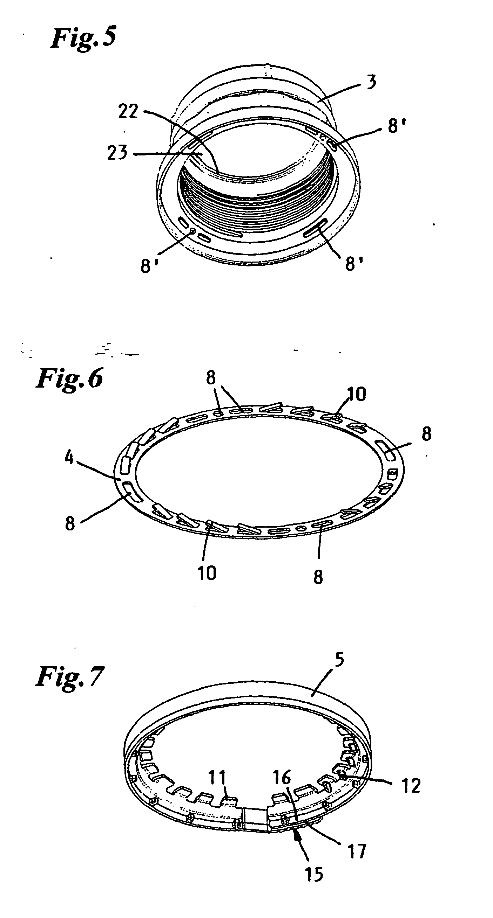

Fig.5 eine perspektivische Ansicht der Verschlusskappe des Behälters nach Fig.1,
Fig.6 eine perspektivische der Unterseite des Klinkenringes nach Fig.3 und
Fig.7 eine perspektivische der Unterseite eines ablösbaren Sicherungsringes.

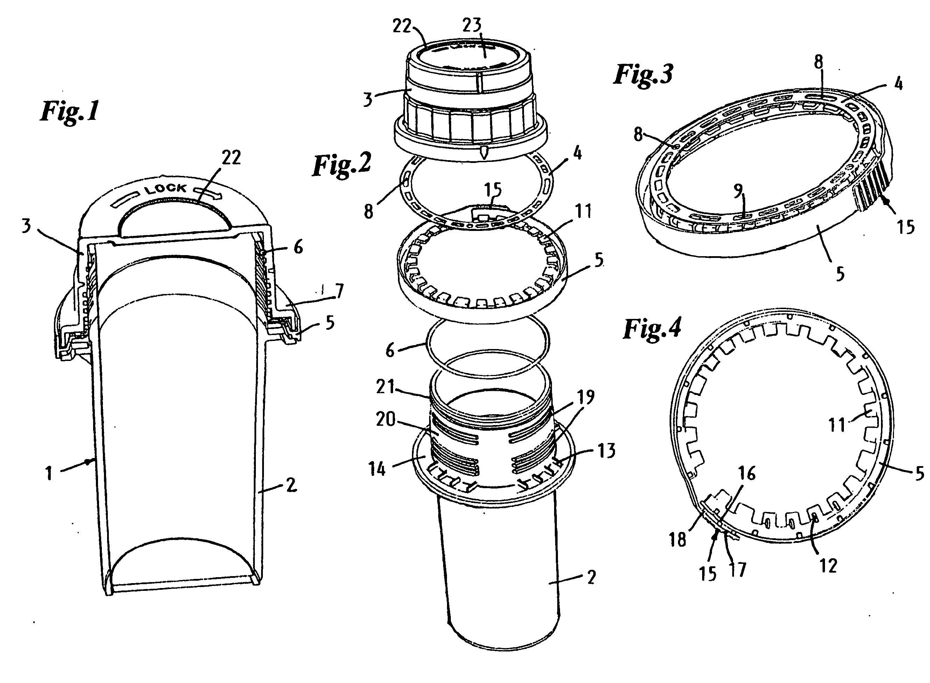

Wie insbesondere die Darstellung der Fig.2 erkennen lässt, besteht der Behälter 1 mit seinem Verschlusssystem aus einem becherförmigen Behälterkörper 2, einer Verschlusskappe 3, einem Klinkenring 4, einem Sicherungsring 5 und einem Dichtring 6.

Der Behälterkörper 2, die Verschlusskappe 3, der Sicherungsring 5 und der Dichtring 6 lassen sich kostengünstig in Kunststoffspritztechnik herstellen. Dabei kann der aus Blech gestanzte Klinkenring 4 durch Anordnung in einer entsprechenden Spritzgiessform, im Bereich einer ringförmig umlaufenden Kappenschulter 7 der Verschlusskappe 3, formschlüssig mit dieser zu einer Einheit verbunden werden. Er kann jedoch auch nach separater Herstellung der Verschlusskappe 3 mit dieser verschweisst werden. Hierzu sind in dem aus Blech ausgestanzten Klinkenring 4 mehrere Eingriffsöffnungen 8 für das Eindringen oder Einfügen und anschliessend verschweissen eines Materialanteils 8' der Verschlusskappe 3 ausgestanzt. Ausserdem werden im gleichen Arbeitsgang durch u-förmige Ausstanzungen 9 federzungenartig schräg nach unten vom Klinkenring 4 und in seiner Umfangsrichtung abstehende Sperrklinken 10 ausgebildet.

Diese Sperrklinken 10 gleiten beim erstmaligen Aufschrauben der Verschlusskappe 3 auf den Behälterkörper 2 federnd über Rastnocken 11, die in Reihe hintereinander an der Oberseite des Sicherungsringes 5 vorgesehen sind. Falls die Sperrklinken 10 in Endposition der Aufschraubbewegung mit ihrer Endkante nicht bereits in eine Lücke zwischen den Rastnocken 11 eingreifen, so erfolgt ein solches Eingreifen sofort beim Versuch, die Verschlusskappe 3 aufzuschrauben.

Ein Mitdrehen des Sicherungsringes 5 mit der Verschlusskappe 3, beim Versuch diese vom Behälterkörper 2 abzuschrauben, wird durch an seiner Unterseite vorgesehene Ankernocken 12 verhindert, die sich in Eingriff mit Sperrnocken 13 des Behälterkörpers 2 befinden. Diese sind beim dargestellten Ausführungsbeispiel an der Oberseite eines kragenförmig umlaufenden Behälterrandes 14 vorgesehen.

Um den Behälter 1 trotz dieser Drehsicherung der Verschlusskappe 3 am Behälterkörper 2 für ein erstmaliges Füllen öffnen zu können, ist der als Garantieverschluss dienende, nach aussen gut sichtbare Sicherungsring 5 vom Behälterkörper 2 ablösbar. Hierzu ist ein am Sicherungsring 5 vorgesehener Solltrennbereich 15 zu zerstören, über den der Sicherungsring 5 geschlossen ist. An diesem Solltrennbereich 15 bildet der Sicherungsring 5 beispielsweise eine Überlappung, deren Teile 16, 17 punktweise über Sollbruchstege 18 miteinander verbunden sind. Der äussere Überlappungsteil 17 bildet dabei eine Griffzunge, die durch eine äussere Riffelung markiert ist.

Nach Füllen des Behälterkörpers 2, beispielsweise durch Urin für eine Dopingkontrolle, lässt sich die Verschlusskappe 3 erneut auf den oberhalb des kragenförmigen Behälterrandes 14 vorgesehenen, Aussengewinde 19 aufweisenden Behälterstutzen 20 aufschrauben. In diesem Fall gelangen die Sperrklinken 10 des mit der Verschlusskappe 3 festverbundenen Klinkenringes 4 erneut in zerstörungsfrei unlösbaren Eingriff, jedoch in diesem Fall mit den am kragenförmigen Behälterrand 14 vorgesehenen Sperrnocken 13. Zur Abdichtung des eingefüllten Behälterinhaltes ist der z.B. als O-Ring ausgebildete Dichtring 6 in einer umlaufenden Rille 21 im äusseren Endbereich des Behälterstutzens 20 vorgesehen und gelangt nach vollständigem Aufschrauben der Verschlusskappe in dichtenden Kontakt mit einer im entsprechenden Bereich leicht konisch verlaufenden Innenfläche der Verschlusskappe, wie es durch die Schnittdarstellung der Fig.1 ersichtlich ist.

Somit hat der Behälter 1 zwei Gruppen (11, 13) von Verriegelungsmitteln, die für den Eingriff mit Verriegelungsmitteln (10) der Verschlusskappe 3 bestimmt sind, von denen eine erste Gruppe (11) an einem am Behälter 1 verankerten und für eine Erstöffnung über einen Solltrennbereich 15 ablösbaren Behälterteil (5) vorgesehen ist und eine zweite Gruppe (13), von dem ablösbaren Behälterteil (5) überdeckt, am Behälter 1 angeformt ist.

Aufgrund der beschriebenen Erfindung wird ein zerstörungsfreies Öffnen des gefüllten Behälters 1 sicher verhindert. Zum Leeren des Behälters 1 ist somit seine Verschlusskappe 3 z.B. durch Aufschneiden zu zerstören. Dies kann durch ein diesem Zweck angepasstes Werkzeug erfolgen, durch das z.B. ein umlaufender Einschnitt entlang eines Absatzes 22 oder entlang einer Rille der Deckelwand 23 ausgeführt wird.

## Patentansprüche

1. Behälter mit einem Verschlusssystem für eine fälschungssichere Aufbewahrung, mit einer mit einem Behälterkörper (2) verschraubten Verschlusskappe (3), wobei zwischen dem Behälter (1) und seiner Verschlusskappe (3) zwei Gruppen (11, 13) von Verriegelungsmitteln vorgesehen sind, von denen eine erste Gruppe (11) an einem am Behälter (1) verankerten und für eine Erstöffnung über einen Solltrennbereich (15) ablösbaren Behälterteil (5) vorgesehen ist und eine zweite Gruppe (13), von dem ablösbaren Behälterteil (5) überdeckt, am Behälterkörper (2) angeformt ist, dadurch gekennzeicchnet, dass die Verschlusskappe (3) sowohl vor dem Erstgebrauch als nach der Befüllung des Behälters (1) in Verschlussposition zerstörungsfrei unlösbar mit dem Behälterkörper (2) verbunden ist und die beiden Gruppen von Verriegelungsmitteln für den Eingriff mit Verriegelungsmitteln (10) der Verschlusskappe (3) bestimmt sind.

2. Behälter nach Anspruch 1, **dadurch gekennzeichnet, dass** die erste Gruppe (11) von Verriegelungsmitteln an einem zwischen der Verschlusskappe (3) und dem Behälterkörper (2) angeordneten, gegen Verdrehung gesperrten und ablösbaren Sicherungsring (5) vorgesehen ist.

3. Behälter nach Anspruch 2, **dadurch gekennzeichnet, dass** der Sicherungsring (5) über einen Solltrennbereich (15) geschlossen ist.

4. Behälter nach Anspruch 2, **dadurch gekennzeichnet, dass** die zweite Gruppe (13) von Verriegelungsmitteln an einem kragenförmig umlaufenden Behälterrand (14) des Behälterkörpers (2) vorgesehen und durch den Sicherungsring (5) überdeckt ist, wobei an der Unterseite des Sicherungsrings (5) vorgesehene Ankernocken (12) mit dieser zweiten Gruppe (13) von Verriegelungsmitteln in Eingriff stehen.

5. Behälter nach Anspruch 1, **dadurch gekennzeichnet, dass** die Verriegelungsmittel der Verschlusskappe (3) in Form von reihenförmig angeordneten Sperrklinken (10) an einem mit der Verschlusskappe (3) fest verbundenen und in ihr eingeschlossenen Klinkenring (4) vorgesehen sind.

6. Behälter nach Anspruch 5, **dadurch gekennzeichnet, dass** der Klinkenring (4) aus Blech geformt ist und die Sperrklinken (10) durch u-förmige Ausstanzungen und Ausbiegungen des Klinkenringes (4) gebildet sind, so dass sie zungenförmig und federnd auslenkbar von der Unterseite des Klinkenringes (4) wegragen.

7. Behälter nach Anspruch 6, **dadurch gekennzeichnet, dass** in dem Klinkenring (4) Eingriffsöffnungen (8) vorgesehen sind, in die ein Materialanteil der Verschlusskappe (3) eingreift.

8. Behälter nach Anspruch 3, **dadurch gekennzeichnet, dass** der Solltrennbereich (15) sich gegenseitig überlappende Teile (16, 17) aufweist, die über mindestens eine Solltrennstelle (18) miteinander verbunden sind, wobei der äussere Teil (17) der Überlappung eine Griffzunge bildet.

9. Behälter nach einem der Ansprüche 1 bis 8, **dadurch gekennzeichnet, dass** der Behälterkörper (2), der Sicherungsring (5) und die Behälterkappe (3) aus Kunststoff geformt sind.

## Claims

1. Container with a closure system for tamper-proof storage, comprising a screw cap (3), screwed with a container body (2), wherein between the container (1) and its screw cap (3), two groups (11, 13) of locking means are provided, of which a first group (11) is provided at a container part (5), which is anchored on the container (1) and is detachable for an initial opening over a predetermined separating region (15), and a second group (13), which is covered by the detachable container part (5) and adapted to the shape of the container body (2), **characterised by** the fact that the screw cap (3) is connected permanently and non-destructively to the container body (2) in the closed position, both prior to initial use and after the container (1) is filled and the two groups of locking means are intended for engaging with the locking means (10) of the screw cap (3).

2. Container according to claim 1, **characterised by** the fact that the first group (11) of locking means is provided at an anti-turn and detachable locking ring (5), which is arranged between the screw cap (3) and the container body (2).

3. Container according to claim 2, **characterised by** the fact that the locking ring (5) is closed over a predetermined separating region (15).

4. Container according to claim 2, **characterised by** the fact that the second group (13) of locking means is provided at a collar-shaped continuous container rim (14) of the container body (2), and is covered by the locking ring (5), wherein anchoring cam (12) that is provided on the underside of the locking ring (5) engages with this second group (13) of locking means.

5. Container according to claim 1, **characterised by** the fact that the locking means of the screw cap (3) in the form of sequential safety catches (10) is provided at a ratchet ring (4), which is connected firmly to the screw cap (3) and enclosed therein.

6. Container according to claim 5, **characterised by** the fact that the ratchet ring (4) is made of sheet metal and the safety catches (10) are formed by U-shaped punching and bending of the ratchet ring (4), so that they protrude in a tongue-shaped and resiliently deflectable manner away from the underside of ratchet ring (4).

7. Container according to claim 6, **characterised by** the fact that access openings (8), into which a substantial portion of the screw cap (3) engages, are provided in the ratchet ring (4).

8. Container according to claim 3, **characterised by** the fact that the predetermined separating region (15) exhibits mutually overlapping parts (16, 17), which are connected to one another via at least one predetermined breaking point (18), wherein the outer part (17) of the overlap forms a gripping tongue.

9. Container according to one of claims 1 to 8, **characterised by** the fact that the container body (2), the locking ring (5) and the container cap (3) are plastic-moulded.

## Revendications

1. Récipient avec un système de fermeture pour une conservation à l'épreuve de la contrefaçon, avec un capuchon de fermeture (3) vissé avec un corps de récipient (2), dans lequel deux groupes (11, 13) de moyens de verrouillage sont prévus entre le récipient (1) et son capuchon de fermeture (3), parmi lesquels un premier groupe (11) est prévu sur une partie de récipient (5) ancrée sur le récipient (1) et détachable par le biais d'une région destinée à la rupture (15) pour une première ouverture et un second groupe (13), recouvert par la partie de récipient détachable (5), est moulé sur le corps de récipient (2), **caractérisé en ce que** le capuchon de fermeture (3) est relié au corps de récipient (2) de manière inamovible sans destruction en position de fermeture aussi bien avant le premier usage qu'après le remplissage du récipient (1) et les deux groupes de moyens de verrouillage sont destinés à l'engagement avec des moyens de verrouillage (10) du capuchon de fermeture (3).

2. Récipient selon la revendication 1, **caractérisé en ce que** le premier groupe (11) de moyens de verrouillage est prévu sur un circlip (5) disposé entre le capuchon de fermeture (3) et le corps de récipient (2), bloqué en rotation et détachable.

3. Récipient selon la revendication 2, **caractérisé en ce que** le circlip (5) est fermé par le biais d'une région destinée à la rupture (15).

4. Récipient selon la revendication 2, **caractérisé en ce que** le second groupe (13) de moyens de verrouillage est prévu sur un bord de récipient périphérique en forme de col (14) du corps de récipient (2) et recouvert par le circlip (5), dans lequel des cames d'ancrage (12) prévues sur le côté inférieur du circlip (5) sont en engagement avec ce second groupe (13) de moyens de verrouillage.

5. Récipient selon la revendication 1 **caractérisé en ce que** les moyens de verrouillage du capuchon de fermeture (3) sont prévus sous la forme de loquets de blocage disposés en rangée (10) sur un anneau de loquets (4) relié de manière fixe au capuchon de fermeture (3) et inclus dans celui-ci.

6. Récipient selon la revendication 5, **caractérisé en ce que** l'anneau de loquets (4) est moulé à partir de tôle et les loquets de blocage (10) sont formés par des découpages et pliures en forme de U de l'anneau de loquets (4) de sorte qu'ils dépassent en s'éloignant du côté inférieur de l'anneau de loquets (4) en forme de languettes et de manière à pouvoir être déviés élastiquement.

7. Récipient selon la revendication 6, **caractérisé en ce que** des ouvertures d'engagement (8) dans lesquelles une portion de matériau du capuchon de fermeture (3) s'engage sont prévues dans l'anneau de loquets (4).

8. Récipient selon la revendication 3, **caractérisé en ce que** la région destinée à la rupture (15) présente des parties se chevauchant mutuellement (16, 17) qui sont reliées ensemble par le biais d'au moins un point destiné à la rupture (18), dans lequel la partie extérieure (17) du chevauchement forme une languette de saisie.

9. Récipient selon l'une quelconque des revendications 1 à 8, **caractérisé en ce que** le corps de récipient (2), le circlip (5) et le capuchon de récipient (3) sont moulés à partir de plastique.
